# EUROPÄISCHE PATENTSCHRIFT

(11) **EP 1 698 286 B1**
(45) Veröffentlichungstag und Bekanntmachung des Hinweises auf die Patenterteilung: **18.11.2009**
(21) Anmeldenummer: 06004135.7
(22) Anmeldetag: 26.03.1999
(51) Int. Cl.: A61B 17/17

(54) **Zielgerät für einen Verriegelungsnagel**
Aiming guide for use with intramedullary nail
Instrument de visée pour clou intramédullaire

(30) Priorität: 09.04.1998 DE 29806564 U
(43) Veröffentlichungstag der Anmeldung: 06.09.2006
(62) Teilanmeldung aus: 99106096.3
(73) Patentinhaber: Stryker Trauma GmbH, 24232 Schönkirchen/Kiel (DE)
(72) Erfinder: Simon, Bernd, 24107 Kiel (DE)
(74) Vertreter: Dilg, Andreas

(56) Entgegenhaltungen:
- EP-A- 0 556 500
- US-A- 4 913 137
- US-A- 4 920 958
- US-A- 5 281 224
- US-A- 5 354 300

## Beschreibung

Die Erfindung bezieht sich auf ein Zielgerät für einen Verriegelungsnagel mit einem ersten Abschnitt, der mit dem zugeordneten Ende des Nagels lösbar verbindbar ist, einem mit dem ersten Abschnitt verbundenen bügelartigen zweiten Abschnitt, der einen Aufnahmeabschnitt aufweist, der annähernd parallel zum Nagel verläuft, wenn der Nagel mit dem ersten Abschnitt verbunden ist und der mindestens eine Querbohrung aufweist für die annähernd passende Aufnahme einer Führungshülse.

In den Unterschenkel- oder Oberschenkelknochen einführbare Verriegelungsnägel weisen zumeist mehrere Querbohrungen auf, durch die hindurch Knochenschrauben geführt werden, um die Verriegelungsnägel sicher im Knochenkanal zu halten. Insbesondere wird dadurch eine Drehsicherung erhalten.

So ist in US 4,920,958 eine u-förmige Kontaktfeder mit Führungslöchern für einen anzusetzenden Knochenbohrer vorgesehen, die am proximalen Ende des Marknagels angeschraubt ist und in einem bestimmten Abstand, analog zum Marknagel, Führungslöscher eingebracht sind.

US 5 281 224 offenbart ein Zielgerät für einen Verrieglungsnagel mit einem mit dem Verrieglungsnagel verbundenen Verbindungssteg und einem Zielkörper, der annähernd parallel zum Verrieglungsnagel verläuft.

Ein besonderes Problem bei Vernegelungsnägeln ist das Auffinden der Querbohrungen beim implantierten Nagel. Hierfür werden daher Zielgeräte verwendet. Eine Kategorie von Zielgeräten arbeitet mit Röntgenstrahlen. Die Querbohrungen des Verriegelungsnagels im Knochen werden auf einem Monitor abgebildet. Ferner findet eine Abbildung eines Zielelements statt. Auf diese Weise ist es möglich, an der Außenseite die Stelle zu markieren, die auf der Achse der Querbohrungen liegt.

Bei einer anderen Kategorie werden Zielgeräte fest mit einem Ende eines Nagels verbunden. Ein bügelartiger Abschnitt weist mindestens eine Durchbohrung auf, deren Achse mit der Achse einer Querbohrung des Nagels ausgerichtet ist, wenn er am Zielgerät angebracht ist. Zur Führung des Bohrwerkzeugs bzw. der Knochenschraube ist auch bekannt, durch die Querbohrung des Zielgerätes eine Führungshülse zu stecken, die bis gegen die Außenseite des Knochens vorgeschoben wird.

Die Führungshülse sitzt zwar passend in der Querbohrung des Zielgerätes, muss jedoch von Hand relativ leicht bewegt werden können, um dem Chirurgen die Arbeit nicht zu erschweren. Dadurch besteht jedoch Gefahr, dass bei der Handhabung des Zielgerätes bzw. beim Bohren und auch beim Einschrauben von Knochenschrauben die Führungshülse nach hinten verrutscht.

Der Erfindung liegt daher die Aufgabe zugrunde, ein Zielgerät für einen Verriegelungsnagel zu schaffen, bei dem die Hülse auf einfache Weise verstellt und in der jeweiligen Position fixiert werden kann.

Diese Aufgabe wird durch die Merkmale des Anspruchs 1 gelöst.

Bei dem erfindungsgemäßen Zielgerät weist der Aufnahmeabschnitt zwei Teile auf, wobei der eine, der einem aufgenommenen Nagel zugekehrt ist, starr mit den übrigen Abschnitten des Zielgeräts verbunden ist, während der zweite Teil gegen Federkraft relativ zum ersten Teil bewegbar ist. Die Querbohrung erstreckt sich durch beide Teile, wobei die Teilbohrungen so angeordnet sind, dass bei entspanntem zweiten Teil die Hülse leicht geklemmt gehalten ist. Wird hingegen der zweite Teil in Richtung des ersten Teils gedrückt, lässt sich die Hülse mehr oder weniger frei in den Teilbohrungen verschieben.

Wird eine Hülse von der Querbohrung des Zielgeräts aufgenommen und in Richtung des aufgenommenen Nagels gedrückt, führt dies zu einer gewissen Verformung des zweiten Teils in Richtung des ersten Teils, wodurch die Hülse gegen einen gewissen Widerstand verschoben werden kann, ohne dass eine zusätzliche Betätigung notwendig ist. Falls hierzu eine zu große Kraft aufzubringen ist, genügt ein leichtes Drücken auf den zweiten Teil, damit die Hülse verschoben werden kann. Hierbei kann die Hülse auch nach hinten geschoben werden, falls sie entfernt werden soll. Wesentlich ist jedoch, dass die Hülse auch mit relativ großem Kraftaufwand nicht aus der Bohrung herausgezogen werden kann, wenn das zweite Teil nicht in Richtung erstes Teil gedruckt wird. Das verschwenkbare zweite Teil wird bei dem Versuch, die Hülse herauszuziehen, vom ersten Teil fort geschwenkt und erhöht die Klemmwirkung. Diese steigt mit aufgebrachter Kraft, wodurch die Hülse gegen ein Zurückschieben sicher gehalten ist.

Es gibt verschiedene Möglichkeiten, die erfindungsgemäße Konstruktion zu verwirklichen. Eine Ausgestaltung sieht hierzu vor, dass erstes und zweites Teil einteilig aus elastischem Werkstoff geformt sind. Es ist natürlich auch denkbar, das zweite Teil am ersten anzulenken und zwischen beiden eine Feder wirken zu lassen.

Eine andere Ausgestaltung sieht vor, dass das zweite Teil am freien Ende des ersten Teils angebracht und von diesem durch einen annähernd parallelen Schlitz getrennt ist.

Das gesamte Zielgerät kann einteilig geformt werden, beispielsweise gegossen werden. Es ist jedoch vorteilhafter, den Aufnahmeabschnitt einteilig und das übrige Gerät einteilig zu formen, da es dann leichter ist, für den Aufnahmeabschnitt einen geeigneten, ausreichend federnden Werkstoff zu wählen. Für die übrigen Abschnitte ist hingegen ein relativ fester und ggf. starrer Werkstoff erforderlich, vor allem wenn das Zielgerät gleichzeitig als Einschlaginstrument dient.

In einem exemplarischen Ausführungsbeispiel wird Zielgerät für einen Verriegelungsnagel mit einem ersten Abschnitt, der mit dem zugeordneten Ende des Nagels lösbar verbindbar ist, einem mit dem ersten Abschnitt verbundenen bügelartigen zweiten Abschnitt, der einen Aufnahmeabschnitt aufweist, der annähernd parallel zum Nagel verläuft, wenn der Nagel mit dem ersten Abschnitt verbunden ist und der mindestens eine Querbohrung aufweist für die annähernd passende Aufnahme einer Führungshülse geschaffen, wobei der Aufnahmeabschnitt aus einem starr mit dem zweiten Abschnitt geformten, dem ersten Abschnitt zugewandten ersten Teil und einen zweiten relativ zum ersten Teil gegen Federkraft bewegbaren äußeren zweiten Teil besteht, wobei sich die Querbohrung durch beide Teile hindurcherstreckt und die Bohrung in den Teilen so gewählt ist, dass bei entspanntem zweiten Teil die Hülse leicht klemmend gehalten ist und bei einer gewissen Verstellung des zweiten Teils auf das erste Teil zu in der Bohrung frei verschiebbar ist.

In einem anderen exemplarischen Ausführungsbeispiel des Zielgeräts sind erstes und zweites Teil einteilig aus einem elastischen Werkstoff geformt.

In einem anderen exemplarischen Ausführungsbeispiel des Zielgeräts ist das zweite Teil am freien Ende des ersten Teils angebracht und von diesem durch einen parallelen Schlitz getrennt.

In einem anderen exemplarischen Ausführungsbeispiel des Zielgeräts ist der Aufnahmeabschnitt einteilig geformt.

In einem anderen exemplarischen Ausführungsbeispiel des Zielgeräts sind erster und zweiter Abschnitt einteilig geformt.

In einem anderen exemplarischen Ausführungsbeispiel des Zielgeräts ist der erste Teil des Aufnahmeabschnitts über Verbindungselemente mit dem zweiten Abschnitt verbunden.

In einem anderen exemplarischen Ausführungsbeispiel des Zielgeräts sind erstes und zweites Teil aus geeignetem Kunststoffmaterial und erster Abschnitt und übriger zweiter Abschnitt aus Metall geformt.

Die Erfindung wird nachfolgend anhand von Ausführungsbeispielen näher erläutert.

Fig. 1 zeigt perspektivisch eine erste Ausführungsform eines Zielgeräts nach der Erfindung.

Fig. 2 zeigt die Seitenansicht einer zweiten Ausführungsform eines Zielgeräts nach der Erfindung.

Fig. 3 zeigt einen Schnitt durch das Zielgerät nach Fig. 2.

Fig. 4 zeigt eine Seitenansicht des Zielgeräts nach Fig. 2 in Richtung Pfeil 4.

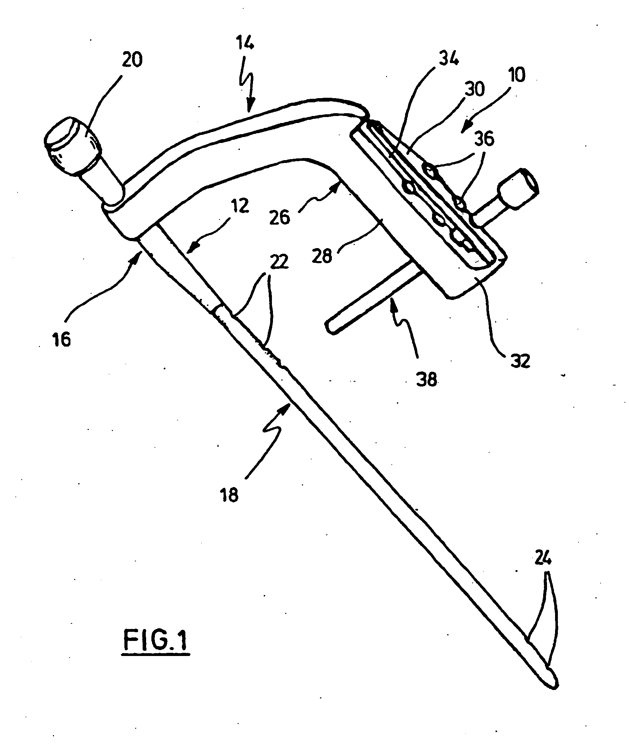

In Fig. 1 ist ein Zielgerät 10 dargestellt, das aus einem ersten Abschnitt 12 und einem bügelartigen zweiten Abschnitt 14 zusammengesetzt ist. Der bügelartige Abschnitt 14 weist am linken Ende eine Durchbohrung auf zur Aufnahme einer Befestigungshülse 16, welche starr in dieser Bohrung befestigt ist. Die Hülse hat am unteren Ende einen Anschlussabschnitt (nicht gezeigt) zur Verbindung mit einem an sich bekannten Verriegelungsnagel 18. Orientierungsmittel des Aufnahmeabschnitts sorgen dafür, dass der Nagel 18 in vorgegebener Drehlage mit der Hülse 16 verbunden wird. Hierzu dient zum Beispiel ein Schraubenbolzen, dessen Kopf bei 20 zu erkennen ist und dessen Gewinde mit einem Innengewindeabschnitt des Nagels 18 zusammenwirkt. Der Verriegelungsnagel 18 weist im oberen Bereich bei 22 Querbohrungen auf und im unteren Bereich Querbohrungen 24. Sie dienen zur Aufnahme von Knochenschrauben (nicht gezeigt), die quer durch den Knochen und durch die Querbohrungen 22, 24 hindurchgeschraubt werden, wie an sich bekannt.

Der Geräteabschnitt 14 weist einen Aufnahmeabschnitt 26 auf, der aus einem ersten Teil 28 und einem zweiten Teil 30 besteht. Das erste Teil ist einteilig mit dem zweiten Abschnitt 14 geformt und starr mit diesem verbunden. Das zweite Teil 30 ist am freien Ende bei 32 an den Abschnitt 28 angebunden und von diesem durch einen annähernd parallel verlaufenden Einschnitt 34 getrennt. Da der Werkstoff, aus dem der Abschnitt 14 geformt ist, federnd ist, kann das Teil 30 auf das Teil 28 zu und von diesem fort verschwenkt bzw. gebogen werden.

Querbohrungen 36 erstrecken sich durch die Teile 28, 30 und dienen zur Aufnahme einer Führungshülse 38, die durch die unterste Bohrung hindurchgesteckt ist. Die Führungshülse 38 kann jeweils annähernd passend von den Bohrungen 36 in den Teilen 28, 30 aufgenommen werden, wobei jedoch die Lage der Bohrungen in den beiden Teilen 28, 30 zueinander derart ist, dass die Hülse in entspannter Lage des Teils 30 leicht klemmend gehalten ist. Wird das Teil 30 ein wenig in Richtung Teil 28 verformt, lässt sich die Hülse 38 leicht in den Bohrungen 36 verschieben. Wird hingegen das Teil 30 in die entgegengesetzte Richtung verformt, wird die Hülse 38 eingeklemmt und kann nicht mehr bewegt werden.

Die Bohrungen 36, insbesondere im Teil 28, sind in ihrer Achse zu den Querbohrungen 22 ausgerichtet. Wird durch die Hülse 38 ein Bohrwerkzeug geführt, bohrt es den Knochen an einer Stelle an, die auf der gemeinsamen Achse einer Querbohrung des Nagels liegt. Für diesen Vorgang wird die Hülse 38 gegen den Knochen vorgeschoben. Ein Zurückschieben der Hülse 38 wird durch die beschriebene Klemmwirkung verhindert. Soll die Hülse gleichwohl entfernt werden, wird das Teil 30 ein wenig gegen das Teil 28 bewegt.

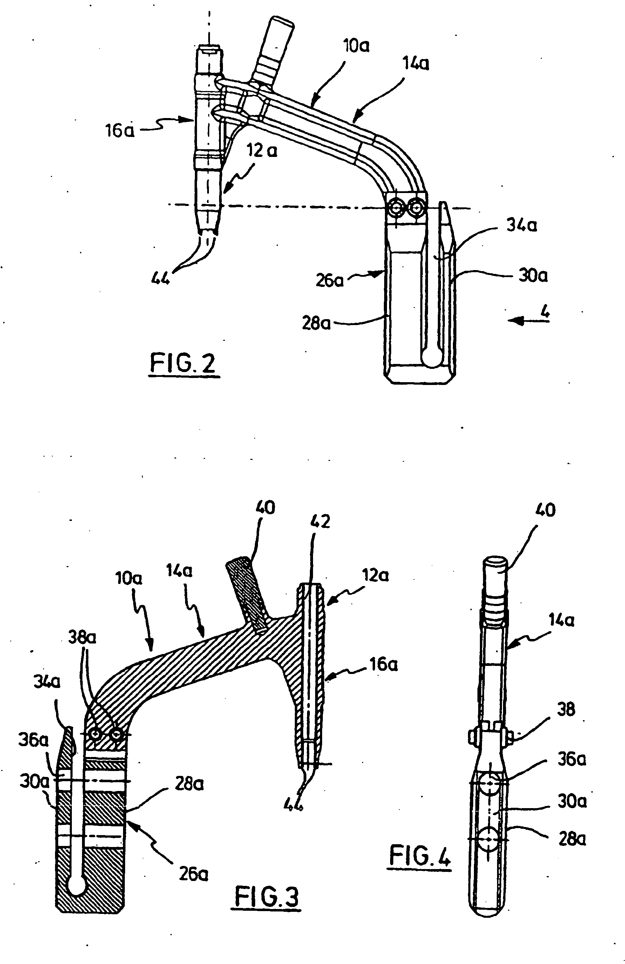

In den Figuren 2 bis 4 ist ein Zielgerät 10a dargestellt, das ähnlich aufgebaut ist wie das nach Fig. 1. Daher werden gleiche Teile mit gleichen Bezugszeichen versehen, denen jedoch ein a hinzugefügt ist.

Man erkennt, dass bei dieser Ausführungsform der Aufnahmeabschnitt 26a über Verbindungshülsen 38a mit dem zweiten Abschnitt 14a verbunden ist. Der Abschnitt 14a ist einteilig mit dem Abschnitt 12a aus einem geeigneten metallischen Werkstoff geformt, während zum Beispiel der Aufnahmeabschnitt 26a aus Kunststoffmaterial besteht. Ansonsten ist der Aufnahmeabschnitt 26a völlig gleich zu dem nach Fig. 1 ausgebildet.

Man erkennt, dass der zweite Abschnitt 14a einen nach oben stehenden Schlagdorn 40 aufweist, der in geeigneter Weise befestigt ist. Man erkennt in Fig. 3, dass der erste Abschnitt 12a eine durchgehende Bohrung 42 aufweist zur Aufnahme eines Befestigungs- bzw. Schraubbolzens zum Anbringen eines Verriegelungsnagels. Zwei Vorsprünge 44 am unteren Ende des Hülsenabschnitts 16a wirken mit nicht gezeigten Ausnehmungen eines Verriegelungsnagels zusammen, um diesem die richtige Orientierung zu geben.

Die lösbare Befestigung des Aufnahmeabschnitts 26a hat den Vorteil, dass verschiedene Aufnahmeabschnitte angebracht werden können je nach eingesetztem Verriegelungsnagel. Muss zum Beispiel im Hinblick auf die Bohrungen 24 des Verriegelungsnagels nach Fig. 1 ein Loch in den Knochen gebohrt werden, bedarf es eines anderen Zielgerätes. Bei der Ausführungsform nach Fig. 2 bis 4 kann daher ein passender Aufnahmeabschnitt 26a an dem übrigen Teil des Zielgerätes angebracht werden.

## Patentansprüche

1. Zielgerät für einen Verriegelungsnagel mit einem ersten Abschnitt, der mit dem zugeordneten Ende des Nagels lösbar verbindbar ist, einem mit dem ersten Abschnitt verbundenen bügelartigen zweiten Abschnitt, der einen Aufnahmeabschnitt aufweist, der mindestens eine Querbohrung aufweist für die annähernd passende Aufnahme einer Führungshülse, wobei der Aufnahmeabschnitt (26, 26a) aus einem starr mit dem zweiten Abschnitt (14a) geformten, dem ersten Abschnitt (16a) zugewandten ersten Teil (28, 28a) und einen zweiten relativ zum ersten Teil (28a) gegen Federkraft bewegbaren äußeren zweiten Teil (30, 30a) besteht, wobei sich die Querbohrung (36, 36a) durch beide Teile (28, 30, 28a, 30a) hindurcherstreckt und die Bohrung (36, 36a) in den Teilen (28, 30, 28a, 30a) so gewählt ist, dass bei entspanntem zweiten Teil (30, 30a) die Hülse (38) leicht klemmend gehalten ist und bei einer gewissen Verstellung des zweiten Teils (30, 30a) auf das erste Teil (28, 28a) zu in der Bohrung (36, 36a) frei verschiebbar ist.

2. Zielgerät nach Anspruch 1, wobei erstes und zweites Teil (28, 30, 28a, 30a) einteilig aus einem elastischen Werkstoff geformt sind.

3. Zielgerät nach Anspruch 2, wobei das zweite Teil (30, 30a) am freien Ende des ersten Teils (28, 28a) angebracht und von diesem durch einen parallelen Schlitz (34, 34a) getrennt ist.

4. Zielgerät nach einem der Ansprüche 1 bis 3, wobei der Aufnahmeabschnitt (26, 26a) einteilig geformt ist.

5. Zielgerät nach einem der Ansprüche 1 bis 4, wobei erster und zweiter Abschnitt (14, 16) einteilig geformt sind.

6. Zielgerät nach einem der Ansprüche 1 bis 4, wobei der erste Teil (28a) des Aufnahmeabschnitts (26a) über Verbindungselemente (38) mit dem zweiten Abschnitt (14a) verbunden ist.

7. Zielgerät nach Anspruch 6, wobei erstes und zweites Teil (28a, 30a) aus geeignetem Kunststoffmaterial und erster Abschnitt (16a) und übriger zweiter Abschnitt (14a) aus Metall geformt sind.

## Claims

1. A target device for an interlocking nail, having a first portion which can be releasably connected with the associated end of the nail, a second, bow-like portion which is connected to the first portion, having a retaining portion, which has at least one through hole to approximately accommodate a guide sleeve, wherein the retaining portion (26, 26a) consists of a first part (28, 28a) which is rigidly formed with the second portion (14a) and which faces the first portion (16a), and an outer second part (30, 30a) which is resiliently movable relative to the first part (28a), wherein the through hole (36, 36a) extends through both parts (28, 30, 28a, 30a) and the hole (36, 36a) in the parts (28, 30, 28a, 30a) is selected so that when the second part (30, 30a) is not under stress, the sleeve (38) is lightly clamped and upon a certain displacement of the second part (30, 30a) towards the first part (28, 28a), it is freely movable in the hole (36, 36a).

2. The target device according to claim 1, wherein the first and second parts (28, 30, 28a, 30a) are unitarily formed from an elastic substance.

3. The target device according to claim 2, wherein the second part (30, 30a) is attached to the free end of the first part (28, 28a) and is separated therefrom by a parallel slot (34, 34a).

4. The target device according to one of claims 1 to 3, wherein the retaining portion (26, 26a) is unitarily formed.

5. The target device according to one of claims 1 to 4, wherein the first and second portions (14, 16) are unitarily formed.

6. The target device according to one of claims 1 to 4, wherein the first part (28a) of the retaining portion (26a) is connected to the second portion (14a) via connecting elements (38).

7. The target device according to claim 6, wherein the first and second parts (28a, 30a) are formed from a suitable plastic material and the first portion (16a) and remaining second portion (14a) are formed from metal.

## Revendications

1. Appareil de visée pour un clou de verrouillage comprenant une première section, qui peut être assemblée de façon amovible avec l'extrémité associée du clou, une seconde section en forme d'étrier qui est assemblée avec la première section et présente une section de réception, pourvue d'au moins un alésage transversal pour le logement approximativement ajusté d'une douille de guidage, la section de réception (26, 26a) étant constituée d'une première partie (28, 28a), formée rigidement avec la seconde section (14a) et tournée vers la première section (16a), et d'une seconde partie extérieure (30, 30a), mobile contre une force de ressort par rapport à la première partie (28a), l'alésage transversal (36, 36a) s'étendant au travers des deux parties (28, 30, 28a, 30a) et l'alésage (36, 36a) dans les deux parties (28, 30, 28a, 30a) étant choisi de sorte que, dans l'état de détente de la seconde partie (30, 30a), la douille (38) est maintenue avec un léger coinçage et peut être librement déplacée dans l'alésage (36, 36a) lors d'un certain déplacement de la seconde partie (30, 30a) sur la première partie (28, 28a).

2. Appareil de visée suivant la revendication 1, dans lequel la première et la seconde parties (28, 30, 28a, 30a) sont formées d'une seule pièce en un matériau élastique.

3. Appareil de visée suivant la revendication 2, dans lequel la seconde partie (30, 30a) est placée sur l'extrémité libre de la première partie (28, 28a) et est séparée de cette dernière par une fente parallèle (34, 34a).

4. Appareil de visée suivant l'une des revendications 1 à 3, dans lequel la section de réception (26, 26a) est formée d'une seule pièce.

5. Appareil de visée suivant l'une des revendications 1 à 4, dans lequel la première et la seconde sections (14, 16) sont formées d'une seule pièce.

6. Appareil de visée suivant l'une des revendications 1 à 4, dans lequel la première partie (28a) de la section de réception (26a) est assemblée avec la seconde section (14a) par l'intermédiaire d'éléments d'assemblage (38).

7. Appareil de visée suivant la revendication 6, dans lequel la première et la seconde parties (28a, 30a) sont formées d'une matière plastique appropriée et la première section (16a) et la seconde section (14a) sont formées de métal.
